Europäisches Patentamt

European Patent Office

Office européen des brevets

(11) Veröffentlichungsnummer: **0 648 502 A1**

## (12) EUROPÄISCHE PATENTANMELDUNG

(21) Anmeldenummer: **94114513.8**

(51) Int. Cl.6: **A61K 39/112**, A61K 39/00

(22) Anmeldetag: **15.09.94**

(30) Priorität: **04.10.93 DE 4333742**

(43) Veröffentlichungstag der Anmeldung:
**19.04.95 Patentblatt 95/16**

(84) Benannte Vertragsstaaten:
**AT BE CH DE DK ES FR GB GR IE IT LI LU MC NL PT SE**

(71) Anmelder: **TAD Pharmazeutisches Werk GmbH**
**Heinz-Lohmann-Strasse 5**
**D-27472 Cuxhaven (DE)**

(72) Erfinder: **Linde, Klaus, Prof. Dr.**
**Bornaische Strasse 137**
**D-04279 Leipzig (DE)**

(74) Vertreter: **Siemons, Norbert, Dr.-Ing. et al**
**Neuer Wall 41**
**D-20354 Hamburg (DE)**

(54) **Lebendimpfstoff mit erhöhter Stabilität.**

(57) 2.1 Die Sicherheit bakterieller Lebend-Impfstämme wird durch Deletionen garantiert, deren Stabilität bezüglich in vivo-repair in der Größenordnung von etwa $10^{-12}$ liegt. Die hohen Anforderungen der Weltgesundheitsorganisation und die weit verbreitete Meinung über die angeblichen Unsicherheiten beim Einsatz von Lebendimpfstoffen unter Praxisbedingungen lassen es für erforderlich erscheinen, den jetzt schon bestehenden Sicherheitspuffer durch eine weitere Erhöhung wenigstens um den Faktor $10^{-7}$ zu vergrößern.

2.2 Die Stabilität eines Lebendimpfstoffes, hergestellt aus mindestens einem Stoffwechseldrift-attenuierten, immunogenen, stabilen Ein- oder Mehrmarker-Lebendimpfstamm mit oder ohne Envelope-Mutation und einem optimalen, der Wirtsspezies-Empfänglichkeit angepaßten Attenuierungsgrad, wird dadurch erhöht, daß als Impfstamm dessen Revertante eingesetzt ist, welche als Suppressormutante noch die Attenuierungs- oder/und Envelope-Marker besitzt.

2.3 Es werden unterschiedliche Varianten des Lebendimpfstoffes sowie eines speziellen Lebendimpfstoffes für Hühner und Kücken und deren Herstellung angegeben.

EP 0 648 502 A1

Die Erfindung betrifft einen Lebendimpfstoff mit erhöhter Stabilität. Gegenüber den bekannten Lebendimpfstoffen ist die Stabilität wenigstens um den Faktor $10^{-7}$ erhöht. Der Impfstoff besteht aus mindestens einem Stoffwechseldrift-attenuierten, immunogenen Lebendimpfstamm mit oder ohne Envelope-Mutation und einem optimal der Wirtsspezies-Empfänglichkeit angepaßten Attenuierungsgrad. Als Wirtsspezies kommen Nutztiere und Menschen infrage. In einer Ausführungsform wird ein Lebendimpfstoff für Kücken und Hühner zur Immunisierung gegen Salmonellen vorgestellt.

Es werden die Merkmale des Lebendimpfstoffes und dessen Herstellung dargestellt.

Die Sicherheit bakterieller Lebend-Impfstämme wird international durch Deletionen garantiert, deren Stabilität in der Größenordnung von etwa $10^{-12}$ liegt (kalkuliert aus dem in vivo repair von Einmarker-Shigellen über genetisch verwandte Coli, nachgewiesen bei Tests an Freiwilligen (Formal et al. sowie DuPont et al.: Immunbiol. Standard; Karger; Basel/München/New York 1971, **15**, 73-78 sowie 213-218). Bei der bekannten Zweimarker (Punktmutation)-Attenuierung resultiert im ungünstigsten Fall eine Gesamtstabilität (als Produkt der Einzelstabilitäten) von wenigstens $10^{-14}$. Entsprechend der Aufgabenstellung dieser Erfindung soll die Gesamtstabilität auf wenigstens $10^{-21}$ erhöht werden, wodurch die Stabilität von (in der Regel nicht üblichen) zwei (an getrennten Genorten gesetzten) attenuierenden Deletionen erreicht wird.

Die hohen Anforderungen der Weltgesundheitsorganisation und die weit verbreitete Meinung über die angeblichen Unsicherheiten beim Einsatz von Lebendimpfstoffen unter Praxisbedingungen lassen es somit für erforderlich erscheinen, den jetzt schon bestehenden Sicherheitspuffer mit einer Stabilität von wenigstens $10^{-14}$ durch eine weitere Erhöhung der Stabilität der Attenuierung auf Werte von wenigstens $10^{-21}$ zu vergrößern und auch die Stabilität der zusätzlich durch einen Envelope-Marker vermittelten antiepidemischen Potenz auf wenigstens $10^{-14}$ anzuheben. Bei der Konstruktion derartiger Lebendimpfstoffe mit einer erhöhten Stabilität soll auf die bewährten und bekannten Prinzipien zurückgegriffen und diese weitergeführt werden.

Bekanntlich besteht eine besondere Schwierigkeit bei der Suche nach wirksamen und verträglichen Impfstämmen darin, ob der Marker-bedingte Attenuierungsgrad ("Stoffwechseldefekt") und die Wirtsspezies charakteristische Empfänglichkeit für den in Frage kommenden Erreger dergestalt miteinander korrelieren, daß

- noch eine begrenzte aber ausreichende in vivo-Vermehrung des Impfstammes möglich ist und demzufolge eine praxisrelevante Immunität resultiert
- bei zu geringer Empfänglichkeit und gleichzeitig relativ starker "Stoffwechselstörung" des Impfstammes eine mehr oder weniger ausgeprägte Überattenuierung besteht und daher (nicht-praxisrelevante) mehrfache Impfstoffgaben notwendig werden, wie z.B. bei S. typhi gal-E 21a
- bei hoher Empfänglichkeit und gleichzeitig relativ geringen "Stoffwechselstörungen" des Impfstammes die "Restvirulenz" unakzeptable Nebenwirkungen auslöst.

Als beinahe klassisches Beispiel für die Wechselbeziehungen zwischen Marker bedingtem Attenuierungsgrad, Wirtsspezies-Empfänglichkeit und Immunogenität ist die Wirksamkeit des Impfstoffes Zoosaloral - Salmonella typhimurium His$^-$ (Attenuierung über Ko-Mutation)/Pur$^-$, i.p. $LD_{50}$ Maus $10^{8.2}$ (Wildstamm etwa $10^1$) cfu - bei Mäusen, Kälbern und Hühnern:

Bezogen auf ein kg Körpergewicht beträgt die parenterale $LD_{50}$ (als Maß für die Empfänglichkeit) bei Mäusen etwa $10^3$, Kälbern etwa $5 \times 10^6$ und Kücken/Hühnern etwa $5 \times 10^8$ Keime. Bei einer einmaligen oralen Immunisierung schützt daher Zoosaloral Mäuse zu > 95 % gegen einen letalen Challenge, Kälber noch praxisrelevant (bei einer $LD_{75}$ Belastung sterben "nur" 36 % der immunisierten Tiere), versagt jedoch wegen Überattenuierung bei Kücken/Hühnern (Linde, K. et al.: Vaccine, 1990, **8**, 278-282).

D.h. der charakteristische Empfänglichkeitsgrad der Wirtsspezies für den jeweiligen Erreger muß durch einen korrespondierenden Attenuierungsgrad ausgeglichen werden, um bei einmaliger Immunisierung eine belastbare Immunität zu erhalten. Dieser an sich logischen (aber offensichtlich nicht gesehene bzw. nicht formulierte) Gesetzmäßigkeit kann erstmals durch Aufdeckung des Prinzips "Attenuierung mittels Stoffwechseldrift - Mutationen" in essentiellen Enzymen und Stoffwechsel-Kompartimenten wie z.B. RNA-Polymerase, Gyrase, Ribosomenproteine (Isolierung über chromosomale z.B. Rifampicin-, Streptomycin-, Nalidixinsäure-Resistenz) - praxisrelevant entsprochen werden. Da bei diesem Prinzip der Logarithmus der (beim empfänglichen Versuchstier gemessenen) $LD_{50}$ linear mit der (verlängerten) Generationszeit korreliert, steht außerdem die (verlängerte) Generationszeit als Attenuierungs-Äquivalent für solche Erreger zur Verfügung, für die ein geeignetes (empfängliches) Versuchstier fehlt. Das Prinzip "Attenuierung mittels Stoffwechseldrift-Mutationen" ist umfassend dargestellt in DD 155294, DD 218834, DD 235828, DD 253182, DD 253184, DD 281118, DD 294420, EP 0263528 sowie in den Publikationen:

- Linde, K.: Zbl. Bakt. Hyg. I. Abt. 1981, **249**, 350-361
- Linde, K.: Dev. Biol. Standard 1983, **53**, 15-28
- Linde, K.: Arch.exper. Vet.med. 1982, **36**, 647-656

- Linde, K. et al.: Arch. exper. Vet.med. 1983, **37**, 353-360
- Linde, K. et al.: Vaccine 1990, **8**, 25-29
- Linde, K. et al.: Vaccine 1990, **8**, 278-282
- Linde, K. et al.: Vaccine 1991, **9**, 101-105
- Linde, K. et al.: Vaccine 1992, **10**, 337-340
- Linde, K. et al.: Vaccine 1993, **11**, 197-200
- Marakusha, B.I. et al.: Z. Microbiol. Epidemiol. Immunol. 1987/4, 3-8

Diese Impfstämme, bei denen einer der attenuierenden Stoffwechseldrift-Marker in der Regel als chromosomale Nalidixinsäure-Resistenz (Gyrase)-Mutation vorliegt, lassen sich durch Hst (high sensitivity to tensides and macrolide antibiotics)-, Rbt (reversion to bile tolerance)- oder Rtt (reversion to tenside tolerance)-Marker optimieren, welche ohne wesentliche Beeinflussung des parenteralen Virulenzverhaltens die Ausscheidung und die Überlebensfähigkeit der Impfstämme in der Außenwelt reduzieren.

Die antiepidemischen Marker sind umfassend in DD 218836, DD 231491, DD 253182, DD 253183, DD 253184 und EP 0263528 sowie in den Publikationen

- Linde, K.: Arch. exper. Vet.med. 1982, **36**, 657-662
- Linde, K.: Dev. Biol. Standard 1983, **53**, 15-28
- Linde, K. et al.: Vaccine 1990, **8**, 278-282

beschrieben.

Schließlich wurde ein Envelope-Marker vorgeschlagen, der die Merkmale der antiepidemischen Marker mit den Merkmalen eines Sicherheits-/und Therapie-Markers verbindet. Der Prototyp eines derartigen Envelope-Markers ist der sogenannte Ssq (supersensitivity to (flour-) quinolones)-Marker, der in der Sm/Rif Stoffwechseldrift-Kombination neben der antiepidemischen Potenz eine Supersensibilität u.a. gegen das derzeit für Salmonellen wirksamste Antibiotikum Ciprofloxacin vermittelt.

Sowohl den unter Verwendung von zwei unabhängig voneinander attenuierenden Stoffwechseldrift-Markern als auch in Kombination von Stoffwechseldrift- und Envelope- bzw. antiepidemischen Markern aufgebauten Impfstämmen ist gemeinsam, daß ihre Reversionshäufigkeit bezüglich den attenuierenden Markern auf einen Wert in der Regel von $\leq 10^{-14}$ und bezüglich der Envelope-Marker bedingten antiepidemischen Potenz ausgewählter Klone auf $\leq 10^{-8}$ beschränkt ist, die unter Praxisbedingungen an sich ausreicht.

Die durch die Erfindung zu lösende Aufgabenstellung besteht demzufolge darin, die Stabilität des zur Herstellung des Impfstoffes dienenden Impfstammes bzw. der -stämme so zu erhöhen, daß hinsichtlich der Attenuierung eine Stabilität von wenigstens $10^{-21}$ und bezogen auf die antiepidemische Potenz von wenigstens $10^{-14}$ erreicht wird. Hierbei sollen bekanntermaßen vorteilhafte Prinzipien für die Einstellung der Attenuierung in Abhängigkeit von der Empfänglichkeit der Wirtsspezies und der Beeinflussung der Ausscheidung und der Überlebensfähigkeit der Impfstämme in der Außenwelt beibehalten und weiter verfeinert werden.

Zur Lösung dieser Aufgabenstellung werden in dem unabhängigen Anspruch 1 ein Lebendimpfstoff, in dem unabhängigen Anspruch 7 ein spezieller Lebendimpfstoff für Hühner und Kücken, in den unabhängigen Ansprüchen 8 und 12 Verfahren zur Herstellung von derartigen Lebendimpfstoffen und in den unabhängigen Ansprüchen 16, 17, 18 und 19 Lebendimpfstämme sowie in den unabhängigen Ansprüchen 20 und 21 deren Verwendung vorgeschlagen.

Anspruch 1 bezieht sich auf einen Lebendimpfstoff, der aus mindestens einem Stoffwechseldrift-attenuierten, immunogenen, stabilen Ein- oder Mehrmarker-Lebendimpfstamm mit oder ohne Envelope-Mutation und einem optimalen, der Wirtsspezies-Empfänglichkeit angepaßten Attenuierungsgrad hergestellt ist. Erfindungsgemäß ist als Impfstamm dessen Revertante eingesetzt, welche als Suppressormutante noch die Attenuierungs- oder/und Envelope-Marker besitzt.

In einer bevorzugten Ausführungsform ist als Impfstamm dessen Generationszeit-verkürzte Revertante eingesetzt, wobei diese Revertante als Suppressormutante einen oder mehrere attenuierende Stoffwechseldrift-Marker besitzt. Die Revertante hat gegenüber dem Ausgangs-Impfstamm einen gleichen oder nur gering verminderten Attenuierungsgrad.

In einer weiteren bevorzugten Ausführungsform ist als Impfstamm dessen Makrolid-tolerante Revertante eingesetzt, wobei diese Revertante als Suppressormutante noch den Envelope-Marker besitzt. Die Revertante hat gegenüber dem Ausgangs-Impfstamm eine gleiche oder gering erhöhte antiepidemische Potenz.

In einer dritten bevorzugten Ausführungsform ist als Impfstamm dessen zweifache Revertante eingesetzt, wobei diese Generationszeit-verkürzte und Makrolid-tolerante Revertante als Suppressormutante sowohl einen oder mehrere attenuierende Stoffwechseldrift-Marker als auch noch den Envelope-Marker besitzt.

Überraschenderweise wurde gefunden, daß ausgehend von Stoffwechseldrift-attenuierten Impfstämmen mit oder ohne zusätzlichem Envelope-Marker die Generationszeit-verkürzten oder/und Makrolid-toleranten Revertanten eine unveränderte Attenuierung und antiepidemische Potenz besitzen, was das Nochvorhandensein der Stoffwechseldrift-Attenuierungs- oder/und Envelope-Marker der Ausgangs-Impfstämme in den Revertanten beweist. Weiterhin hat sich gezeigt, daß beim Übergang von den Ausgangs-Impfstämmen zu deren Revertanten/Suppressormutanten die Attenuierung im Sinne einer Anpassung an die Wirtsspezies-Empfänglichkeit noch in geringem Maße variiert werden kann. Gleiches trifft für die antiepidemische Potenz zu.

Beispielhaft für den vorstehend beanspruchten Lebendimpfstoff werden erfindungsgemäß in dem unabhängigen Anspruch 7 nachfolgend aufgeführte Salmonella-Impfstämme für einen Lebendimpfstoff zur oralen Immunisierung von Kücken sowie oralen oder parenteralen Immunisierung/Boosterung von Hühnern gegen Salmonella-Infektionen vorgeschlagen, die von Ausgangs-Impfstämmen mit einer Generationszeit von etwa 31 bis 32 Minuten und/oder einer MHK für Erythromycin von $\leq$ 5 $\mu$g/ml abgeleitet wurden. Die Generationszeit-verkürzten Revertanten

- S.tm Ssq/Sm 60/Rif 42/-GVR II 30
  Generationszeit etwa 26,0 Minuten
- S.ent Ssq/Sm 24/Rif 12/-GVR II 30
  Generationszeit etwa 27,0 Minuten
- S.inf Ssq/Sm 153/Rif 7/-GVR II 30
  Generationszeit etwa 25,0 Minuten
- S.ana Ssq/Sm 81/Rif 21/-GVR II 30
  Generationszeit etwa 26,5 Minuten

weisen eine Generationszeit von bis zu $\leq$ 27 Minuten auf. Bei den Makrolid-toleranten Revertanten

- S.tm Ssq-MTR 16/Sm 60/Rif 42
- S.ent Ssq-MTR 25/Sm 24/Rif 12
- S.inf Ssq-MTR 12/Sm 153/Rif 7
- S.ana Ssq-MTR 23/Sm 81/Rif 21

wurde die MHK für Erythromycin von $\leq$ 5 $\mu$g/ml auf $\geq$ 20 $\mu$g/ml angehoben.

Die aufgeführten Revertanten/Suppressormutanten wurden von den in der DSM - Deutsche Sammlung von Mikroorganismen und Zellkulturen GmbH, D-38124 Braunschweig hinterlegten Salmonella-Impfstämme abgeleitet:

| | |
|---|---|
| . S.tm Ssq/Sm 60/Rif 42 | DSM 8433 |
| . S.ent Ssq/Sm 24/Rif 12 | DSM 8435 |
| . S.inf Ssq/Sm 153/Rif 7 | DSM 8434 |
| . S. ana Ssq/Sm 81/Rif 21 | DSM 8441 |

Die erfindungsgemäß vorgeschlagenen Revertanten/Suppressormutanten repräsentieren eine neue Generation von erhöht stabilen (Gesamtstabilität = Produkt aus den Markern der Ausgangs-Impfstämme und der Suppressormutation) Impfstämmen. Sie nutzen die überraschend gefundene Gesetzmäßigkeit, daß Reversionen/Suppressormutationen zum Pseudowildtyp mit unverändertem oder nur gering vermindertem Attenuierungsgrad und/oder unveränderter oder nur gering variierender antiepidemischer Potenz um Größenordnungen häufiger sind als Rückmutationen. Es wurde untersucht, wie weit dies sich auch zur Prüfung der tatsächlichen Stabilität der Stoffwechseldrift-Attenuierungs- und Envelope-Marker nutzen und zugleich zur Gewinnung einer neuen Generation von extrem stabilen Impfstämmen verwenden läßt, welche die von der WHO postulierten Stabilitäts-/und Sicherheitsforderungen ideal erfüllen (Report of a WHO Scientific Group: Oral Enteric Bacterial Vaccines, World Health Organization, Technical Report Series No. 500, 1972) und die eine Impfstoff-Einführung erschwerenden Diskussionen über die Stabilität von Zweimarker-Impfstämmen bei diesen nunmehr Dreimarker-attenuierten Impfstämmen ad absurdum führt. Die Sicherheit bakterieller Lebend-Impfstämme wird international durch Deletionen garantiert (s.o.), deren Stabilität in der Größenordnung von etwa $10^{-12}$ liegt (kalkuliert aus dem in vivo repair von Einmarker-Shigellen über genetisch verwandte Coli, nachgewiesen bei Tests an Freiwilligen. Formal et al. sowie DuPont et al.: Immunbiol. Standard; Karger; Basel/München/New York 1971, **15**, 73-78 sowie 213-218). Bei der verwendeten Zweimarker (Punktmutation)-Attenuierung resultiert im ungünstigsten Fall eine Gesamtstabilität (als Produkt der Einzelstabilitäten) von wenigstens $10^{-14}$. Eine hinzukommende Suppressormutation erhöht somit die Gesamtstabilität auf wenigstens $10^{-21}$ und erreicht damit die Stabilität von (in der Regel nicht üblichen) zwei (an getrennten Genorten gesetzten) attenuierenden Deletionen. Bei Kopplung von zwei attenuierenden Stoffwechseldrift-Markern mit einer Envelope-Mutation und nachfolgender Herstellung der Suppressormutation von beiden Prinzipien würde sogar --- da laut WHO-Definition eine die Impfstamm-Infektionsketten unterbrechende Mutation als Attenuierungsmarker gilt (WHO Technical Report Series No. 500, 1972) --- eine an sich nicht notwendige Gesamtstabilität von wenigstens $\leq 10^{-35}$ resultieren.

In Abhängigkeit von den Markern werden zwei Arten von Revertanten unterschieden und im folgenden ausgeführt.

## Generationszeit-verkürzte Revertanten (GVR)

Die Stabilität der Stoffwechseldrift (Antibiotika-Resistenz-Mutation)-Attenuierung mit einer entsprechenden (bei Salmonella-Impfstämmen für Kücken/Hühner von 22 auf 31 - 32 Minuten angehobenen) Generationszeitverlängerung, läßt sich nur indirekt über die erhaltene Resistenz und die definiert verlängerte Generationszeit belegen.

Langsam wachsende Stoffwechseldrift-attenuierte Impfstämme bieten aber für solche Klone einen Selektionsvorteil, bei denen weitere Mutationen (da es eine genetische Stabilität an sich nicht gibt) über "korrigierte" Stoffwechselabläufe zu kürzeren Generationszeiten führen. D.h. mit einer Anreicherung von GVR-Stämmen ist unter den entsprechenden Selektionsdrücken in einer langsamer wachsenden Impfstamm-Population zu rechnen.

Die vom bekannten Wissensstand ausgehende Schlußfolgerung "Generationszeit-Verlängerung führt zur Attenuierung" und somit "Generationszeit-Verkürzung (umgekehrt proportional) zur Virulenz-Anhebung" wird nun in Kenntnis um die Bedeutung von Reversionen/Suppressormutationen (welche um Größenordnungen häufiger auftreten als Rückmutationen) widerlegt (Linde, K. Arch. exper. Vet. med. 1978, **32**, 943).

In dem folgenden Beispiel 1 wird am bewährten Modell S.typhimurium/Maus der direkte Beweis geführt, daß die GVR (bezogen auf den Ausgangs-Impfstamm) in ihrer Attenuierung unverändert oder nur gering vermindert sind und demzufolge noch ihre ursprünglichen Attenuierungsmarker besitzen.

Diese Generationszeit-verkürzten Revertanten/Suppressormutanten repräsentieren somit einen neuen Typ von extrem stabilen Impfstämmen, deren Reversionshäufigkeit höchstens $\leq 10^{-21}$ beträgt (Gesamtstabilität = Produkt der Einzelstabilitäten (bei Annahme einer unwahrscheinlich hohen Größenordnung von $\leq 10^{-7}$ pro Einzelmarker): Sm x Rif x Suppressormutation).

## Makrolid-tolerante Revertanten (MTR)

Die Reversionshäufigkeit der Envelope-Mutation, gemessen an der Zahl von 20 $\mu$g Erythromycintoleranten Klonen, liegt in der Regel zwischen $10^{-6}$ und $10^{-8}$ und nur bei wenigen ausgewählten Stämmen bei $\leq 10^{-8}$.

Es wurde gefunden, daß die Makrolid-toleranten Klone - trotz der wiedererlangten Wildstamm-Toleranz für Erythromycin sowie in der Regel dem Verlust der Supersensibilität u.a. für Ciprofloxacin, Doxycyclin, Chloramphenicol - in ihrer antiepidemischen Funktion (reduzierte Überlebensfähigkeit in der Außenwelt und verkürzte Ausscheidung bei Kücken) etwa dem Ausgangs-Impfstamm entsprechen oder nur gering um dessen Werte variieren und demzufolge die ursprüngliche Envelope-Mutation noch besitzen.

Einzelheiten sind dem folgenden Beispiel 2 zu entnehmen.

Diese Makrolid-toleranten Revertanten/Suppressormutanten repräsentieren somit einen neuen Typ von extrem stabilen Impfstämmen, deren Reversionshäufigkeit in der antiepidemischen Potenz höchstens $10^{-14}$ beträgt (Gesamtstabilität = Produkt aus ursprünglichem Envelope-Marker und Suppressormutation).

Somit führen die von Stoffwechseldrift-attenuierten Einmarker- oder Mehrmarker-Ausgangs-Impfstämmen mit oder ohne zusätzlichem antiepidemischen Envelope-Marker abgeleiteten Generationszeitverkürzten und/oder Makrolid-toleranten Revertanten zu Impfstämmen, die in hohem Maße den strengen Anforderungen an die Benutzung von Lebendimpfstoffen unter Praxisbedingungen gerecht werden.

Die Aufgabe zur Herstellung eines Lebendimpfstoffes mit erhöhter Stabilität wird gemäß Anspruch 8 gelöst unter Verwendung von mindestens einem Stoffwechseldrift-attenuierten, immunogenen, stabilen Ein- oder Mehrmarker-Lebendimpfstamm mit oder ohne Envelope-Mutation und einem optimalen, der Wirtsspezies-Empfänglichkeit angepaßten Attenuierungsgrad. Erfindungsgemäß wird, ausgehend von einem Stoffwechseldrift-attenuierten Ausgangs-Impfstamm ohne oder mit zusätzlicher Envelope-Mutation und einer an die Wirtsspezies angepaßten Attenuierung und zugleich definiert verlängerter Generationszeit, die Generationszeitverkürzte oder/und Makrolid-tolerante Revertante isoliert. Diese Suppressormutante, welche noch die Attenuierungs- oder/und Envelope-Marker des Ausgangs-Impfstammes besitzt, wird als erhöht stabiler Impfstamm eingesetzt, aus dem mit an sich bekannten Verfahren der Lebendimpfstoff hergestellt wird.

In weiterer Ausgestaltung des erfindungsgemäßen Verfahrens können gegebenenfalls mittels der Suppressormutante gegenüber dem Ausgangs-Impfstamm die bestimmenden Eigenschaften noch beeinflußt werden.

Bei stammabhängig bevorzugter Abspaltung einer Generationszeitverkürzten Revertante mit etwas geringerem Attenuierungsgrad wird zur Isolierung einer optimal attenuierten Suppressormutante ein Ausgangs-Impfstamm mit höherer (gegebenenfalls bis zur Grenze der Überattenuierung) Attenuierung eingesetzt.

Zur Herstellung eines Impfstoffes mit einer erhöhten Stabilität in der antiepidemischen Potenz wird eine Revertante mit einer gegenüber dem Ausgangs-Impfstamm gleichen oder gering erhöhten antiepidemischen Potenz eingesetzt.

Durch Anwendung dieses Verfahrens in beliebiger Reihenfolge wird von einem Stoffwechseldrift-attenuierten Ausgangs-Impfstamm mit Makrolid-Sensibilitäts-(Envelope)-Marker eine sowohl Generationszeit-verkürzte als auch Makrolid-tolerante Revertante erhalten.

Beispielhaft für das vorstehend beschriebene Verfahren wird zur Herstellung eines optimal auf Kücken oder Hühner abgestimmten Impfstoffes erfindungsgemäß eines der folgenden Verfahren vorgeschlagen.

Es wird zur Herstellung des Impfstoffes ein erhöht stabiler Impfstamm ausgewählt, dessen Ausgangs-Impfstamm gegenüber dem Wildstamm eine Verlängerung der Generationszeit von etwa 22 Minuten auf etwa 31 bis 32 Minuten hat, die sich bei der Generationszeit-verkürzten Revertante auf eine Generationszeit von bis zu ≤ 27 Minuten verkürzt.

Ein weiteres Verfahren ist dadurch gekennzeichnet, daß ein Impfstamm mit erhöhter Stabilität in der antiepidemischen Potenz ausgewählt wird, dessen Ausgangs-Impfstamm eine Makrolid-Sensibilität besitzt, die sich bei der Makrolid-toleranten Revertante in einer Anhebung der MHK für Erythromycin von ≤ 5 $\mu$g/ml auf ≥ 20 $\mu$g/ml äußert.

Schließlich können beide Verfahren auch in beliebiger Reihenfolge miteinander kombiniert werden, indem von einem Stoffwechseldrift-attenuierten Ausgangs-Impfstamm mit Makrolid-Sensibilitäts-(Envelope)-Marker eine sowohl Generationszeitverkürzte als auch Makrolid-tolerante Revertante zur Herstellung eines Lebendimpfstoffes mit erhöhter Stabilität ausgewählt wird.

Als Impfstamm wird einer oder mehrere der im Anspruch 7 beanspruchten Impfstämme eingesetzt.

Im folgenden wird der Lebendimpfstoff und das Verfahren zur Herstellung des Lebendimpfstoffes in Beispielen näher beschrieben.

**Material und Methoden**

Verwendete Stämme
- Wildstämme
  Salmonella (S.) typhimurium, S. enteritidis, S. infantis, S. anatum
- Sets von Stoffwechseldrift-Zwei-(Drei)marker (Gesamtstabilität als Produkt der Einzelstabilitäten)-Impfstämmen mit zusätzlichem Envelope-Marker und graduell abgestufter Attenuierung/graduell verlängerter Generationszeit (GT) (als Attenuierungs-Äquivalent) sowie weitere hinterlegte Impfstämme wie z.B.

| | |
|---|---|
| . S. typhimurium Ssq/Sm 60/Rif 42 Hinterlegungsnummer: DSM 8433 | GT: etwa 31 Minuten |
| . S. enteritidis Ssq/Sm 24/Rif 12 Hinterlegungsnummer: DSM 8435 | GT: etwa 32 Minuten |
| . S. infantis Ssq/Sm 153/Rif 7 Hinterlegungsnummer: DSM 8434 | GT: etwa 31 Minuten |
| . S. anatum Ssq/Sm 81/Rif 21 Hinterlegungsnummer: DSM 8441 | GT: etwa 32 Minuten |
| . S. typhimurium Nal 2/Rif 9/Rtt Hinterlegungsnummer: DSM 8432 | GT: etwa 32 Minuten |
| (Hinterlegungsstelle: DSM-Deutsche Sammlung von Mikroorganismen und Zellkulturen GmbH Braunschweig) | |

- Verwendete Versuchstiere und Nutztiere zur Prüfung der jeweiligen Mutanten der Sets mit an sich bekannten Methoden
  . Hähnchen-Kücken (Brauneileger, gegen Marek geimpft)
  . ICR- und Prob 01-Mäuse

**Beispiel 1**

**Generationszeit-verkürzte Revertanten (GVR)**, abgeleitet von Stoffwechseldrift-attenuierten Salmonella Ausgangs-Impfstämmen (mit Envelope-Marker) für Kücken/Hühner, als Impfstämme mit erhöhter Stabilität in der Attenuierung

Die Ausgangs-Impfstämme (mit Envelope-Marker) werden in 50 ml Nährbouillon (oder ein anderes beliebiges flüssiges Medium) geimpft und dann fortlaufend mit dem Verdünnungsfaktor $\leq$ 1:1000 passagiert.

**Markerkontrollen:**

Nach jeweils vier Passagen wird mittels Impfstrich das erhaltene Wachstum auf Nähragar mit (12,5 $\mu$g Nalidixinsäure/ml oder) 200 $\mu$g Streptomycin/ml sowie 200 $\mu$g Rifampicin/ml (sowie das fehlende Wachstum bei 20 $\mu$g Erythromycin/ml) geprüft.

Innerhalb der 30 kontrollierten Passagen waren die Marker stabil.

**Generationszeit (GT)-Bestimmung im MS-2 Testsystem** (Abbott):

In flüssigen Kulturen (Nähr-, LB- und Minimalmedium-Bouillon) sind die GT bis zur etwa fünften Passage nicht meßbar verändert. Ab sechster Passage reichern sich in den Ausgangs-Impfstamm-Populationen (GT etwa 31 bis 32; Wildstamm etwa 22 Minuten) schneller wachsende GVR-Klone an (stufenweise Verkürzung der GT). Dieser Prozeß verläuft in den getrennten Ansätzen mit unterschiedlicher Schnelligkeit. Die GT zwischen der 10. bis 15. Passage differieren zwischen 29 und 27 Minuten, bis zur 30. Passage können GT von $\leq$ 25 Minuten auftreten. Bei weiteren Passagen sind Klone mit noch kürzeren GT wahrscheinlich.

Anmerkung: Auf festen Medien bleiben die GT bis zur geprüften 20. Passage unverändert (geringere Zahl an Teilungsschritten (Überimpfung mit Öse) = Ausverdünnung auf Populationsniveau oder anderer Selektionsdruck?).

**Unveränderte oder nur gering verminderte Attenuierungshöhe von S.typhimurium GVR-Stämmen** im Vergleich zum Ausgangs-Impfstamm:

- Ausgangs-Impfstamm und GVR-Stämme: Je fünf Mäuse (Prob 01: Versuchsstation der Universität Leipzig) erhalten i.p. $10^5$, $10^6$ und $10^7$ cfu
- Wildstamm: Je fünf Mäuse erhalten i.p. $10^3$ und $10^5$ cfu.

Die im Zeitraum von zwei Wochen gestorbenen Tiere werden ermittelt.

| S. typhimurium GVR-Stämme | Passagen | GT (min) | †/15 Mäusen | |
|---|---|---|---|---|
| Nal 2/Rif 9-GVR: Mm A 11/Rtt | 11 | 28,0 | 3 | Wh 3 |
| GVR: Mm C 11/Rtt | 11 | 28,5 | 1 | |
| GVR: Mm E 11/Rtt | 11 | 27,0 | 7 | |
| GVR: Nb A 15/Rtt | 15 | 29,0 | 4 | |
| GVR: Nb B 15/Rtt | 15 | 28,5 | 7 | Wh 4 |
| GVR: Nb B 25/Rtt | 25 | 27,0 | 2 | |
| GVR: Nb C 15/Rtt | 15 | 28,5 | 0 | Wh 0 |
| GVR: Nb D 15/Rtt | 15 | 28,5 | 2 | |
| GVR: Nb D 25/Rtt | 25 | 28,0 | 1 | |
| GVR: Nb E 15/Rtt | 15 | 28,5 | 2 | |
| GVR: LB A 15/Rtt | 15 | 28,5 | 3 | |
| GVR: LB B 15/Rtt | 15 | 28,5 | 5 | |
| GVR: LB C 15/Rtt | 15 | 28,0 | 7 | Wh 4 |
| GVR: LB C 25/Rtt | 25 | 26,0 | 7 | |
| GVR: LB D 15/Rtt | 15 | 28,5 | 0 | Wh 0 |
| GVR: LB D 25/Rtt | 25 | 26,5 | 0 | |
| GVR: LB E 15/Rtt | 15 | 27,0 | 7 | Wh 4 |
| *Ssq/Sm 60/Rif 42-GVR III 12 | 12 | 29,0 | 7 | |
| GVR III 16 | 16 | 28,0 | 8 | |
| GVR I 30 | 30 | 28,5 | 8 | |
| GVR II 30 | 30 | 26,0 | 9 | |
| Nal 2/Rif 9/Rtt drei parallele Teste | | 32,0 | 4 | Wh 6, 7 |
| Prototyp vier Teste 1992-1990 | | | 5, 8, 5, 6 | |

Wildstamm-Kontrolle: i.p. $10^3$ cfu = $\geq$ 14 ; i.p. $10^5$ cfu = 15

Mm: flüssiges Minimalmedium

Nb: Nährbouillon

LB: LB-Bouillon

Wh: Tierversuch mit gleichem Stamm wiederholt

* S.tm Ssq/Sm 60/Rif 42 Ausgangsstamm; GT: 31 Minuten

Bis auf einzelne offensichtlich etwas höher (oder gering weniger) attenuierte Klone weisen die untersuchten GVR-Stämme in ihrem Attenuierungsverhalten Streubreiten auf, welche mit der Schwankungsbreite der in sieben getrennten Ansätzen 1993-1990 ermittelten Werte des Ausgangs-Impfstammes übereinstimmen und innerhalb der versuchsbedingten Varianz liegen. Selbst wenn einzelne GVR-Stämme gering weniger attenuiert sind, ist dies für die Praxis irrelevant: Die $LD_{50}$- Maus Differenzen zum Wildstamm (i.p. $LD_{50}$ Maus etwa $10^1$ cfu) von etwa fünf logarithmischen Stufen ist gewahrt.

Bei stammabhängig bevorzugter Abspaltung von GVR mit evtl. deutlich geringerem (und unter Umständen nicht zu akzeptierbaren Nebenwirkungen führenden) Attenuierungsgrad greift man zur Isolierung optimal attenuierter Suppressormutanten vorteilhaft auf Ausgangs-Impfstämme mit höherer (bis zur Grenze der Über-) Attenuierung zurück.

M.a.W.: Die GVR sind (wie Sm-id- und T-tol-Stämme, Linde, K.: Arch.exper. Vet.med. 1978, **32**, 943-949) keine Rückmutanten, sondern offensichtlich intra- und intergenetische Suppressormutanten, welche noch ihre Attenuierungsmarker besitzen und deren Attenuierungspegel um den Ausgangswert des Impfstammes variiert.

Zum Nachweis der im Vergleich zu den Ausgangs-Impfstämmen unveränderten Invasivität erhielten $\leq$ 36 Stunden alte Kücken oral $10^9$ cfu der GVR. Nach fünf und acht Tagen wurde die Keimzahl/Gramm Leber quantitativ (s. Beispiel 2) sowie über die Anreicherung (Nährmedien mit je 100 $\mu$g Streptomycin und Rifampicin/ml bei den GVR der Ssq/Sm/Rif-Stämme oder 100 $\mu$g Rifampicin und 12,5 $\mu$g Nalidixinsäure/ml bei den GVR vom Nal/Rif/Rtt-Stamm) qualitativ bestimmt: Die Keimzahlen der GVR in der Kücken-Leber entsprechen etwa den Werten der Ausgangs-Impfstämme. Bei S.infantis und S.anatum werden sowohl beim Wildstamm, Ausgangs-Impfstamm als auch bei dem ausgewählten GVR im Vergleich zu S.enteritidis und S.typhimurium geringere Keimzahlen gefunden, was mit der Beobachtung von Methner, U. (Dissertation, Universität Leipzig, Veterinärmedizinische Fakultät, 1991) korreliert, wonach S. infantis für Hühner eine verminderte Invasivität besitzt.

Somit bieten sich Generationszeit-verkürzte Revertanten, wie z.B. S.tm Ssq/Sm 60/Rif 42/-GVR II 30, S.ent Ssq/Sm 24/Rif 12/-GVR II 30, S.inf Ssq/Sm 153/Rif 7/-GVR II 30 und S.ana Ssq/Sm 81/Rif 21/-GVR II 30, als Salmonella Impfstämme mit extremer Stabilität in der Attenuierung an (Gesamtstabilität der virulenzreduzierenden Attenuierungsmarker mindestens $\leq 10^{-21}$: Sm x Rif x Suppressormutation). (Siehe auch Tabelle am Ende)

## Beispiel 2

**Makrolid-tolerante Revertanten (MTR)**, abgeleitet vom Ssq (Envelope)-Marker der Sm/Rif Stoffwechseldrift-attenuierten Impfstämme für Kücken/Hühner, als Salmonella-Impfstämme mit erhöhter Stabilität der antiepidemischen Potenz

$10^8$ bis $10^9$ cfu der Ssq/Sm/Rif Ausgangs-Impfstämme werden auf Nähragar mit 20 $\mu$g Erythromycin/ml (oder im Zweischritt-Verfahren zuerst auf 5 bis 10 $\mu$g Erythromycin/ml und von dieser Kultur dann auf 20 $\mu$g Erythromycin/ml) gespatelt und die gewachsenen Kolonien/Klone nach etwa zehn Nähragar-Passagen auf erhaltene Makrolid-Toleranz geprüft. Die Toleranz/Sensibilität dieser Makrolid-toleranten Revertanten für Na-Desoxycholat, Na-Dodecylsulfat sowie u.a. Ciprofloxacin (als derzeit für Salmonellen wirksamstes Antibiotikum), Chloramphenicol und Doxycyclin entspricht in der Regel den Wildstamm-Werten, einzelne Revertanten von S. enteritidis besitzen jedoch noch die bis zu vierfach höhere (Super-) Sensibilität des Ausgangs-Impfstammes für Ciprofloxacin.

Die Absterbekinetik ($10^6$ cfu/ml Startkeimzahl) in Wasser bei 37°C (als indirektes Maß für die Außenwelt-Resistenz) zeigt nicht die Wildstamm-Werte, sondern variiert (im Vergleich zum Wildstamm/Impfstamm ohne Envelope-Marker) um die auf etwa zwei Drittel verkürzte Überlebensdauer des Impfstammes.

Eine Auswahl dieser Revertanten - hinsichtlich der gleichen oder noch kürzeren Überlebensdauer in Wasser wie der Ausgangs-Impfstamm und bei S.enteritidis der verbliebenen Supersensibilität für Ciprofloxacin - wurde am Kückenmodell im Vergleich mit dem Ausgangs-Impfstamm auf Invasivitäts- und Ausscheidungsverhalten getestet. Hierzu erhielten $\leq$ 36 Stunden alte Kücken oral $10^9$ cfu der jeweiligen Ausgangs-Impfstämme und der von ihnen abgeleiteten Makrolid-toleranten Revertanten:

- Nachweis der Invasivität: Nach fünf und acht Tagen wurde die Keimzahl/Gramm Leber quantitativ (Leberhomogenat in 5 ml PBS, 3 x 0,1 ml gespatelt; Nachweisgrenze $\leq$ 20 Keime/Gramm) sowie über die Anreicherung in Nährbouillon mit Antibiotika-Zusatz (s. Beispiel 1) qualitativ bestimmt.

- Prüfung des Ausscheidungs-Verhalten: Über einen Zeitraum von 12 Tagen werden im Faeces die Salmonella-Keimzahlen als Promille-Anteil der Coli-Besiedlung gemessen und diese Werte mit den Daten eines zusätzlich mitgeführten Vergleichs-Impfstammes S.tm Nal 2/Rif 9 ohne Envelope-Marker (mit der gleichen i.p. $LD_{50}$ Maus und etwa gleicher Generationszeit-Verlängerung wie z.B. der S.tm Ssq/Sm 60/Rif 42) in Beziehung gesetzt.

Auch in diesem Modell zeigten die Makrolid-toleranten Revertanten ein etwa gleiches Verhalten wie der Ausgangs-Impfstamm (mit Envelope-Marker);

- Invasivität: Die Keimzahlen der Revertanten in der Kücken-Leber entsprechen den Werten des Ausgangs-Impfstammes. Bei S.infantis und S.anatum werden sowohl beim Wildstamm, Ausgangs-Impfstamm als auch bei den ausgewählten Revertanten im Vergleich zu S. enteritidis und S.typhimurium geringere Keimzahlen gefunden, was allerdings mit der Beobachtung von Methner, U. (Dissertation Universität Leipzig, Veterinärmedizinische Fakultät, 1991) korreliert, wonach S.infantis für Hühner eine verminderte Invasivität besitzt.

- Ausscheidungs-Verhalten: Das quantitative Ausscheidungsverhalten der Makrolid-toleranten Revertanten entspricht, gemessen am Promille-Anteil der Enterobakterien, etwa dem der Ausgangs-Impfstämme und sinkt innerhalb von fünf bis sechs Tagen unter die 0,1 Promille-Grenze.

M.a.W.: Das bei den geprüften Makrolid-toleranten Revertanten im Vergleich zu den Ausgangs-Impfstämmen gefundene etwa gleiche, teils sogar raschere Absterben in Wasser, sowie das de facto identische Ausscheidungsverhalten (und die unveränderte Invasivität) bei Kücken, beweist indirekt, daß es sich bei diesen Stämmen nicht um Rückmutanten im Envelope-Marker handelt und daher der Ssq-Marker in den Ausgangs-Impfstämmen eine um Größenordnungen höhere Stabilität aufweist als die auf Nähragar mit $\leqq 10^{-8}$ im Stammpaß angegebene Reversions-Häufigkeit.

Somit bieten sich die Makrolid-toleranten Revertanten, wie z.B. S.tm Ssq-MTR 16/Sm 60/Rif 42, S.ent Ssq-MTR 25/Sm 24/Rif 12, S.inf Ssq-MTR 12/Sm 153/Rif 7 und S.ana Ssq-MTR 23/Sm 81/Rif 21, als Impfstämme mit erhöhter Stabilität in der antiepidemischen Potenz an (Gesamtstabilität = Produkt aus den Ssq-Marker und Suppressormutation Einzelstabilitäten). (s. folgende Tabelle)

Anhang: Tabelle zu Beispiel 1 und 2

Von Stoffwechseldrift-attenuierten Zweimarker (Ausgangs)-Impfstämmen mit Envelope-Marker abgeleitete GVR sowie MTR[*]: De facto unveränderte Invasivität (ø Keimzahl/Gramm Leber fünf und acht Tage nach oraler Gabe von $10^9$ cfu an $\leq$ 36 Stunden Kücken); verbliebene antiepidemische Potenz der MTR[*] (Absterbekinetik von $10^6$ cfu/ml in Wasser bei 37°C)

| Salmo-nella | Stämme | | GT (min) | ø Keimzahl/Gramm Leber | | cfu Wasser |
|---|---|---|---|---|---|---|
| | | | | fünfte Tag | achte Tag | neunte Tag |
| typhi-murium | Wildstamm | | 22,0 | $6{,}0 \times 10^3$ | nt | $4 \times 10^5$ |
| | Ssq/Sm 60/Rif | 42 | 31,0 | $1{,}5 \times 10^3$ | $2{,}8 \times 10^2$ | $4 \times 10^3$ |
| | GVR | II/30 | 26,0 | $2{,}5 \times 10^3$ | $6{,}3 \times 10^2$ | |
| | GVR | III/16 | 28,0 | $1{,}5 \times 10^3$ | $2{,}8 \times 10^2$ | |
| | MTR | 16 | | $1{,}0 \times 10^3$ | $1{,}4 \times 10^2$ | $1 \times 10^4$ |
| | MTR | 40 | | $1{,}5 \times 10^3$ | $5{,}6 \times 10^2$ | $1 \times 10^4$ |
| enteri-tidis | Wildstamm | | 22,0 | $5{,}0 \times 10^3$ | nt | $3 \times 10^5$ |
| | Ssq/Sm 24/Rif | 12 | 32,0 | $4{,}0 \times 10^3$ | nt | $2 \times 10^4$ |
| | GVR | II/30 | 28,0 | $4{,}5 \times 10^3$ | $5{,}3 \times 10^2$ | |
| | GVR | IV/16 | 27,0 | $2{,}0 \times 10^3$ | nt | |
| | MTR | 2 | | $2{,}5 \times 10^3$ | $1{,}9 \times 10^2$ | $5 \times 10^3$ |
| | MTR | 25 | | $2{,}5 \times 10^3$ | $5{,}6 \times 10^2$ | $7 \times 10^3$ |
| anatum | Wildstamm | | 22,0 | $1{,}5 \times 10^2$ | nt | $4 \times 10^4$ |
| | Ssq/Sm 81/Rif | 21 | 32,0 | $3{,}5 \times 10^1$ | Anrei- | $1 \times 10^4$ |
| | GVR | II/30 | 26,5 | $1{,}0 \times 10^2$ | cherung | |
| | GVR | III/16 | 27,5 | $2{,}0 \times 10^2$ | meist | |
| | MTR | 5 | | $3{,}5 \times 10^1$ | positiv | $1 \times 10^4$ |
| | MTR | 23 | | $7{,}0 \times 10^1$ | | $1 \times 10^4$ |
| infan-tis | Wildstamm | | 22,0 | $2{,}0 \times 10^2$ | nt | $5 \times 10^5$ |
| | Ssq/Sm 153/Rif | 7 | 31,0 | $1{,}0 \times 10^2$ | Anrei- | $6 \times 10^4$ |
| | GVR | II/30 | 25,0 | $7{,}0 \times 10^1$ | cherung | |
| | GVR | III/24 | 28,5 | $1{,}0 \times 10^2$ | meist | |
| | MTR | 12 | | $2{,}0 \times 10^2$ | positiv | $7 \times 10^4$ |
| | MTR | 28 | | $2{,}0 \times 10^2$ | | $6 \times 10^4$ |

[*] MTR: Promotionsarbeiten der AiP Adler, T. und Jaensch, C. (1993/94)

**Patentansprüche**

1. Lebendimpfstoff mit einer erhöhten Stabilität, hergestellt aus mindestens einem Stoffwechseldrift-attenuierten, immunogenen, stabilen Ein- oder Mehrmarker-Lebendimpfstamm mit oder ohne Envelope-Mutation und einem optimalen, der Wirtsspezies-Empfänglichkeit angepaßten Attenuierungsgrad, dadurch gekennzeichnet, daß als Impfstamm dessen Revertante eingesetzt ist, welche als Suppressormutante noch die Attenuierungs- oder/und Envelope-Marker besitzt.

EP 0 648 502 A1

**2.** Lebendimpfstoff nach Anspruch 1, dadurch gekennzeichnet, daß als Impfstamm dessen Generationszeit-verkürzte Revertante eingesetzt ist, wobei diese Revertante als Suppressormutante einen oder mehrere attenuierende Stoffwechseldrift-Marker besitzt.

**3.** Lebendimpfstoff nach Anspruch 1, dadurch gekennzeichnet, daß als Impfstamm dessen Makrolid-tolerante Revertante eingesetzt ist, wobei diese Revertante als Suppressormutante noch den Envelope-Marker besitzt.

**4.** Lebendimpfstoff nach Anspruch 1 und 2, dadurch gekennzeichnet, daß die Revertante gegenüber dem Ausgangs-Impfstamm einen gleichen oder gering verminderten Attenuierungsgrad hat.

**5.** Lebendimpfstoff nach Anspruch 1 und 3, dadurch gekennzeichnet, daß die Revertante gegenüber dem Ausgangs-Impfstamm eine gleiche oder gering erhöhte antiepidemische Potenz hat.

**6.** Lebendimpfstoff nach Anspruch 1 bis 5, dadurch gekennzeichnet, daß als Impfstamm dessen zweifache Revertante eingesetzt ist, wobei diese Generationszeit-verkürzte und Makrolid-tolerante Revertante als Suppressormutante sowohl einen oder mehrere attenuierende Stoffwechseldrift-Marker als auch noch den Envelope-Marker besitzt.

**7.** Salmonella-Lebendimpfstoff zur oralen Immunisierung von Kücken sowie oralen oder parenteralen Immunisierung/Boosterung von Hühnern gegen Salmonella-Infektionen, hergestellt aus mindestens einem attenuierten immunogenen Impfstamm, insbesondere nach einem der Ansprüche 1 bis 6, dadurch gekennzeichnet, daß der Impfstoff aus einem oder mehreren der
- Generationszeit-verkürzten Revertanten mit einer von etwa 31 bis 32 Minuten auf bis zu ≤ 27 Minuten verkürzten Generationszeit
  . S.tm Ssq/Sm 60/Rif 42/-GVR II 30
    Generationszeit etwa 26,0 Minuten
  . S.ent Ssq/Sm 24/Rif 12/-GVR II 30
    Generationszeit etwa 27,0 Minuten
  . S.inf Ssq/Sm 153/Rif 7/-GVR II 30
    Generationszeit etwa 25,0 Minuten
  . S.ana Ssq/Sm 81/Rif 21/-GVR II 30
    Generationszeit etwa 26,5 Minuten
- Makrolid-toleranten Revertanten mit einer von ≤ 5 $\mu$g auf ≥ 20 $\mu$g Erythromycin/ml angehobenen MHK
  . S.tm Ssq-MTR 16/Sm 60/Rif 42
  . S.ent Ssq-MTR 25/Sm 24/Rif 12
  . S.inf Ssq-MTR 12/Sm 153/Rif 7
  . S.ana Ssq-MTR 23/Sm 81/Rif 21
besteht.

**8.** Verfahren zur Herstellung eines Lebendimpfstoffes mit einer erhöhten Stabilität, hergestellt aus mindestens einem Stoffwechseldrift-attenuierten, immunogenen, stabilen Ein- oder Mehrmarker-Lebendimpfstamm mit oder ohne Envelope-Mutation und einem optimalen, der Wirtsspezies-Empfänglichkeit angepaßten Attenuierungsgrad, dadurch gekennzeichnet, daß ausgehend von einem Stoffwechseldrift-attenuierten Ausgangs-Impfstamm ohne oder mit zusätzlicher Envelope-Mutation und einer an die Wirtsspezies angepaßten Attenuierung und zugleich definiert verlängerter Generationszeit die Generationszeit-verkürzte oder/und Makrolid-tolerante Revertante isoliert wird und diese Revertante als Suppressormutante mit den Attenuierungs- oder/und Envelope-Marker(n) des Ausgangs-Impfstammes als Impfstamm verwendet wird, um mit an sich bekannten Verfahren einen Lebendimpfstoff herzustellen.

**9.** Verfahren nach Anspruch 8, dadurch gekennzeichnet, daß bei stammabhängig bevorzugter Abspaltung einer Generationszeitverkürzten Revertante mit geringerem Attenuierungsgrad zur Isolierung einer optimal attenuierten Suppressormutante ein Ausgangs-Impfstamm mit höherer (gegebenenfalls bis zur Grenze der Überattenuierung) Attenuierung eingesetzt wird.

**10.** Verfahren nach Anspruch 8, dadurch gekennzeichnet, daß zur Herstellung eines Impfstoffes mit einer erhöhten Stabilität in der antiepidemischen Potenz eine Revertante mit einer gegenüber dem Aus-

12

gangs-Impfstamm gleichen oder gering erhöhten antiepidemischen Potenz eingesetzt wird.

11. Verfahren nach Anspruch 8 bis 10, dadurch gekennzeichnet, daß von einem Ausgangs-Impfstamm mit Makrolid-Sensibilitäts-(Envelope)-Marker eine sowohl Generationszeit-verkürzte als auch Makrolid-tolerante Revertante eingesetzt wird.

12. Verfahren nach einem der Ansprüche 8 und 9 zur Herstellung eines optimal auf Kücken oder Hühner abgestimmten Impfstoffes, dadurch gekennzeichnet, daß ein erhöht stabiler Impfstamm ausgewählt wird, dessen Ausgangs-Impfstamm gegenüber dem Wildstamm eine Verlängerung der Generationszeit von etwa 22 Minuten auf etwa 31 bis 32 Minuten hat, die sich bei der Generationszeit-verkürzten Revertante auf eine Generationszeit von bis zu $\leq$ 27 Minuten verkürzt.

13. Verfahren nach einem der Ansprüche 8 und 10 zur Herstellung eines optimal auf Kücken oder Hühner abgestimmten Impfstoffes, dadurch gekennzeichnet, daß ein Impfstamm mit erhöhter Stabilität in der antiepidemischen Potenz ausgewählt wird, dessen Ausgangs-Impfstamm eine Makrolid-Sensibilität besitzt, die sich bei der Makrolid-toleranten Revertante in einer Anhebung der MHK für Erythromycin von $\leq$ 5 $\mu$g auf $\geq$ 20 $\mu$g/ml äußert.

14. Verfahren nach Anspruch 12 und 13, dadurch gekennzeichnet, daß von einem Ausgangs-Impfstamm mit Makrolid-Sensibilitäts-(Envelope)-Marker eine sowohl Generationszeit-verkürzte als auch Makrolid-tolerante Revertante ausgewählt wird.

15. Verfahren nach einem der Ansprüche 12 bis 14, dadurch gekennzeichnet, daß einer oder mehrere der Impfstämme gemäß Anspruch 7 eingesetzt werden.

16. Salmonella-Impfstämme erhöhter Stabilität aus einer oder mehreren Suppressormutante(n) mit verkürzten Generationszeiten von bis zu $\leq$ 27 Minuten

17. Salmonella-Impfstämme erhöhter Stabilität aus einer Suppressormutante mit einer MHK von $\leq$ 5 $\mu$g auf $\geq$ 20 $\mu$g Erythromycin/ml angehobenen Makrolid-Toleranz

18. Salmonella-Impfstämme erhöhter Stabilität aus Suppressormutanten mit verkürzter Generationszeit und Makrolid-Toleranz

19. Salmonella-Impfstämme nach einem der Ansprüche 16 bis 18 S.tm Ssq/Sm 60/Rif 42/-GVR II 30, S.ent Ssq/Sm 24/Rif 12/-GVR II 30, S.inf Ssq/Sm 153/Rif 7/-GVR II 30, S.ana Ssq/Sm 81/Rif 21/-GVR II 30, S.tm Ssq-MTR 16/Sm 60/Rif 42, S.ent Ssq-MTR 25/Sm 24/Rif 12, S.inf Ssq-MTR 12/Sm 153/Rif 7 und/oder S.ana Ssq-MTR 23/Sm 81/Rif 21 und deren Varianten in dem angegebenen Bereich der Generationszeit bzw. der Makrolid-Toleranz

20. Verwendung der Impfstämme nach einem der Ansprüche 1 bis 7 als Lebendimpfstoffe mit erhöhter Stabilität

21. Verwendung der Impfstämme nach Anspruch 7 als Lebendimpfstoff zur oralen Immunisierung von Kücken sowie oralen oder parenteralen Immunisierung/Boosterung von Hühnern gegen Salmonella-Infektionen

| | **EINSCHLÄGIGE DOKUMENTE** | | EP 94114513.8 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int Cl.6) |
|---|---|---|---|
| A | WO - A - 92/11 361<br>(THE GENERAL HOSPITAL CORPORATION)<br>   * Zusammenfassung;<br>    Ansprüche *<br>        -- | 1,7,8 | A 61 K 39/112<br>A 61 K 39/00 |
| A | WO - A - 91/06 317<br>(WASHINGTON UNIVERSITY)<br>   * Zusammenfassung;<br>    Ansprüche *<br>        -- | 7,8 | |
| A | US - A - 4 837 151<br>(STOCKER)<br>   * Zusammenfassung;<br>    Ansprüche *<br>        -- | 7,8 | |
| D,A | EP - A - 0 263 528<br>(VEB SÄCHSISCHES SERUMWERK)<br>   * Zusammenfassung;<br>    Ansprüche *<br>        -- | 7,8 | **RECHERCHIERTE SACHGEBIETE (Int. Cl.6)** |
| A | WO - A - 86/03 123<br>(THE BOARD OF TRUSTEES OF THE LELAND STANFORD JR.UNIVERISTY)<br>   * Zusammenfassung;<br>    Ansprüche *<br>        ---- | 7,8 | A 61 K<br>C 12 N |

Der vorliegende Recherchenbericht wurde für alle Patentansprüche erstellt.

| Recherchenort<br>WIEN | Abschlußdatum der Recherche<br>20-12-1994 | Prüfer<br>SCHNASS |
|---|---|---|